(19) Europäisches Patentamt
European Patent Office
Office européen des brevets

(11) **EP 3 950 710 A1**

(12) **EUROPEAN PATENT APPLICATION**
published in accordance with Art. 153(4) EPC

(43) Date of publication:
**09.02.2022 Bulletin 2022/06**

(21) Application number: 20784191.7

(22) Date of filing: **25.03.2020**

(51) International Patent Classification (IPC):
**C07K 14/78** (2006.01)  **C12M 3/00** (2006.01)
**C12N 5/071** (2010.01)  **C12N 5/09** (2010.01)

(52) Cooperative Patent Classification (CPC):
**C07K 14/78; C12M 3/00**

(86) International application number:
**PCT/JP2020/013436**

(87) International publication number:
**WO 2020/203582 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.04.2019 JP 2019069976**

(71) Applicants:
• **TOPPAN INC.**
**Tokyo 110-0016 (JP)**

• **Osaka University**
**Suita-shi, Osaka 565-0871 (JP)**

(72) Inventors:
• **KITANO Shiro**
**Tokyo 110-0016 (JP)**
• **IRIE Shinji**
**Tokyo 110-0016 (JP)**
• **MATSUSAKI Michiya**
**Suita-shi, Osaka 565-0871 (JP)**

(74) Representative: **TBK**
**Bavariaring 4-6**
**80336 München (DE)**

(54) **EXTRACELLULAR MATRIX-CONTAINING COMPOSITION AND METHOD FOR PRODUCING SAME, AND THREE-DIMENSIONAL TISSUE CONSTRUCT AND METHOD FOR PRODUCING SAME**

(57)     The present invention relates to an extracellular matrix-containing composition containing a fragmented extracellular matrix component and a compound bound or adsorbed to the fragmented extracellular matrix component.

EP 3 950 710 A1

**Description**

**Technical Field**

The present invention relates to an extracellular matrix-containing composition, a method for producing the same, a three-dimensional tissue construct, and a method for producing the same.

**Background Art**

As a method for artificially producing a structure imitating a living tissue, for example, a method for producing a three-dimensional tissue construct by culturing coated cells in which the entire surface of cultured cells are covered with an adhesive membrane (Patent Literature 1), a method for producing a three-dimensional tissue construct including three-dimensionally arranging cells coated with a collagen-containing coating to form a three-dimensional tissue construct (Patent Literature 2), a method for producing a three-dimensional tissue construct including forming coated cells having a coating formed on the surface of cells and three-dimensionally arranging the coated cells, wherein the formation of the coated cells includes immersion of cells in a liquid containing a coating component and separating the immersed cells and the liquid containing a coating component by a liquid permeable membrane (Patent Literature 3), a method for producing a three-dimensional cell tissue including mixing cells with a cationic substance and an extracellular matrix component to obtain a mixture, and collecting cells from the obtained mixture to form cell aggregates on a substrate (Patent Literature 4) and the like are known.

In addition, the inventors have proposed a method for producing a three-dimensional tissue construct having a high collagen concentration by bringing cells into contact with endogenous collagen and preferably bringing cells into contact with fibrous exogenous collagen (Patent Literature 5). These three-dimensional tissue constructs are expected to be used as substitutes for laboratory animals, transplant materials, and the like.

Incidentally, in a living body, extracellular matrixes such as collagen interact with each other to form a tissue. In addition, it has been suggested that, in order for extracellular matrixes to interact with each other, the extracellular matrixes are bound and/or adsorbed to a lower molecular-weight-compound, and the bound and/or adsorbed compound plays an important role in the extracellular matrix interaction (for example, Non-Patent Literature 1-3).

**Citation List**

**Patent Literature**

[Patent Literature 1] Japanese Unexamined Patent Publication No. 2012-115254
[Patent Literature 2] PCT International Publication No. WO2015/072164
[Patent Literature 3] PCT International Publication No. WO2016/027853
[Patent Literature 4] PCT International Publication No. WO2017/146124
[Patent Literature 5] PCT International Publication No. WO2018/143286

**Non-Patent Literature**

[Non-Patent Literature 1] M. C. Erat et al., Proc. Natl. Acad. Sci. USA 2009, 106, 4195.
[Non-Patent Literature 2] Y. Tatara et al., Glycobiology 2015,25,557.
[Non-Patent Literature 3] M. Shawn et al., Proc. Natl. Acad. Sci. USA 1998, 95, 7275.

**Summary of Invention**

**Technical Problem**

According to the above method for producing a three-dimensional tissue construct, a three-dimensional tissue construct can be artificially and easily produced depending on the method, but it is required to produce a three-dimensional tissue construct that can imitate an interaction of various compounds in an actual living body more reliably.

One aspect of the present invention is to provide an extracellular matrix-containing composition that can form a three-dimensional tissue construct that is closer to a state in vivo. Another aspect of the present invention is to provide a three-dimensional tissue construct that is closer to a state in vivo and a method for producing the same.

**Solution to Problem**

That is, the present invention relates to, for example, the following inventions.

[1] An extracellular matrix-containing composition, comprising a fragmented extracellular matrix component and a compound bound or adsorbed to the fragmented extracellular matrix component.

[2] The extracellular matrix-containing composition according to [1], wherein the fragmented extracellular matrix component comprises a fragmented collagen component.

[3] The extracellular matrix-containing composition according to [1] or [2], wherein the average length of the fragmented extracellular matrix component is 100 nm or more and 200 $\mu$m or less.

[4] The extracellular matrix-containing composition according to any one of [1] to [3], wherein the compound is at least one selected from the group consisting of heparan sulfate, chondroitin sulfate, and fibronectin.

[5] A method for producing an extracellular matrix-containing composition, including a process of bringing a fragmented extracellular matrix component into contact with a compound that is able to be bound or adsorbed to the fragmented extracellular matrix component.

[6] The method for producing an extracellular matrix-containing composition according to [5], wherein the fragmented extracellular matrix component is obtained by fragmenting an extracellular matrix component in an aqueous medium.

[7] The method for producing an extracellular matrix-containing composition according to [5] or [6], wherein the fragmented extracellular matrix component contains a fragmented collagen component.

[8] A method for producing a three-dimensional tissue construct, including: a first process in which the extracellular matrix-containing composition according to any one of [1] to [4] is brought into contact with cells in an aqueous medium; and a second process in which the cells in contact with the extracellular matrix-containing composition are cultured.

[9] The method for producing a three-dimensional tissue construct according to [8], including a process in which, in the aqueous medium, the fragmented extracellular matrix component, a compound bound or adsorbed to the fragmented extracellular matrix component, and the cells are precipitated, after the first process and before the second process.

[10] The method for producing a three-dimensional tissue construct according to [8] or [9], wherein the first process is performed after a layer of the cells is formed in the aqueous medium.

[11] The method for producing a three-dimensional tissue construct according to any one of [8] to [10], wherein the cells contain extracellular matrix producing cells.

[12] The method for producing a three-dimensional tissue construct according to any one of [8] to [11], wherein the cells are one or more types of cells selected from the group consisting of vascular endothelial cells, cancer cells, cardiomyocytes, smooth muscle cells, and epithelial cells.

[13] The method for producing a three-dimensional tissue construct according to any one of [8] to [12], wherein a total content of the fragmented extracellular matrix component and the compound based on a total mass of the fragmented extracellular matrix component, the compound and the cells is 10 mass% or more and 90 mass% or less.

[14] A three-dimensional tissue construct containing cells and the extracellular matrix-containing composition according to any one of [1] to [4], wherein at least some of the cells are in contact with the fragmented extracellular matrix component.

**Advantageous Effects of Invention**

According to the present invention, it is possible to provide an extracellular matrix-containing composition that can form a three-dimensional tissue construct that is closer to a state in vivo. According to the present invention, it is possible to provide a three-dimensional tissue construct that is closer to a state in vivo and a method for producing the same. When the extracellular matrix-containing composition of the present invention is used, for example, it is possible to obtain a three-dimensional tissue construct that imitates the existing state of blood vessels and cells around blood vessels. In addition, when an extracellular matrix-containing composition of the present invention is used, it is possible to obtain a three-dimensional tissue construct having a coarser blood vessel density than in the related art.

**Brief Description of Drawings**

FIG. 1 shows microscopic images of fibrillated collagen components to which various compounds are bound or adsorbed.

FIG. 2 is a microscopic image of a fibrillated collagen component to which a fluorescein-labeled chondroitin sulfate is bound or adsorbed.

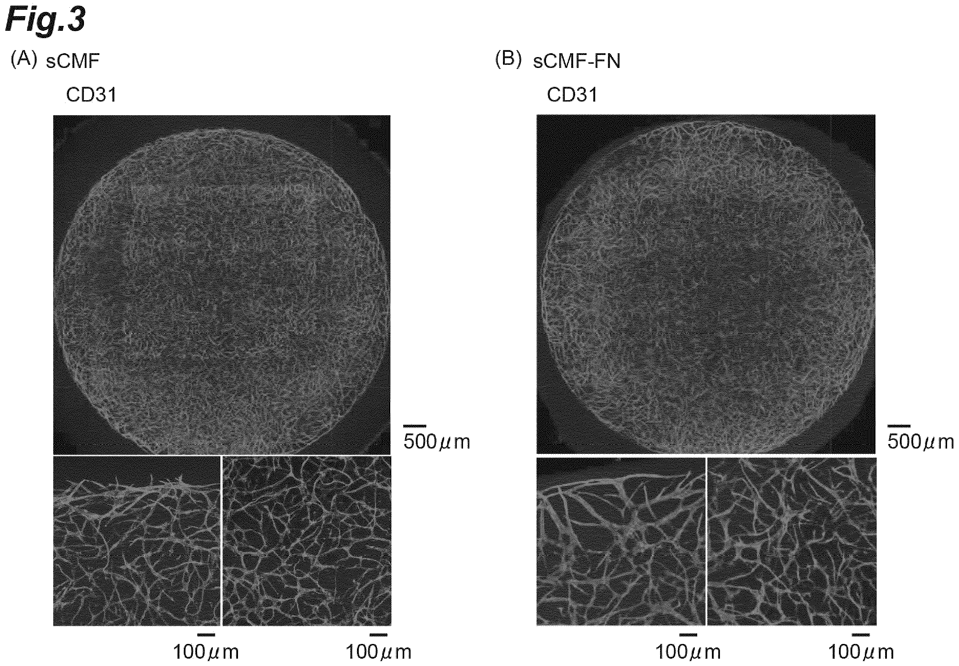

FIG. 3 shows images of observation results of capillary vessels composed of three-dimensional tissue constructs with CD31 staining.

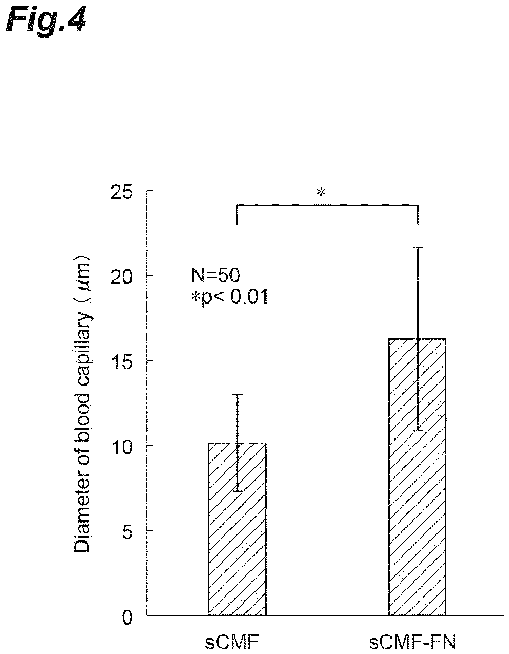

FIG. 4 is a graph showing the measurement results of the diameter of capillary vessels.

FIG. 5 shows images of observation results of capillary vessels composed of three-dimensional tissue constructs with vimentin staining and CD31 staining.

FIG. 6 shows images of observation results of three-dimensional tissue constructs with toluidine blue (TB) staining.

FIG. 7 is a graph showing measurement results of the length of nuclei of cells in a three-dimensional tissue construct.

FIG. 8 shows images of observation results of three-dimensional tissue constructs with CD31 staining.

**Description of Embodiments**

Hereinafter, forms for implementing the present invention will be described in detail. However, the present invention is not limited to the following embodiments.

<Extracellular matrix-containing composition>

An extracellular matrix-containing composition according to the present embodiment includes a fragmented extracellular matrix component (fragmented extracellular matrix component) and a compound bound or adsorbed to the fragmented extracellular matrix component.

The extracellular matrix-containing composition according to the present embodiment can be suitably used as a scaffolding material or the like for forming a three-dimensional tissue construct.

The extracellular matrix component is a material that fills gaps between at least some cells in the three-dimensional tissue construct. The extracellular matrix component is an extracellular matrix molecule aggregate formed of a plurality of extracellular matrix molecules. The extracellular matrix molecule may be a substance existing outside cells in an organism. As the extracellular matrix molecules, any substance can be used as long as it does not adversely affect the growth of cells and formation of cell aggregates. Examples of extracellular matrix molecules include collagen, laminin, fibronectin, vitronectin, elastin, tenascin, entactin, fibrillin, and proteoglycan, but the present invention is not limited thereto. The extracellular matrix components may be used alone or in combination. The extracellular matrix component may contain, for example, collagen, or may be composed of collagen. When the extracellular matrix component contains collagen, collagen functions as a scaffold for cell adhesion, and formation of a three-dimensional cell structure is further promoted. Here, the extracellular matrix molecule may be a modifier or variant of the above extracellular matrix molecule as long as it does not adversely affect the growth of cells and formation of cell aggregates, and may be a polypeptide such as a chemically synthesized peptide. The extracellular matrix molecule may have a repetition of a sequence represented by Gly-X-Y, which is a characteristic of collagen. Here, Gly represents a glycine residue, and X and Y each independently represent an arbitrary amino acid residue. A plurality of Gly-X-Y's may be the same as or different from each other. When a repetition of a sequence represented by Gly-X-Y is provided, there are few restrictions on the arrangement of molecular chains, and the function as a scaffolding material is further improved. In the extracellular matrix molecule having a repetition of a sequence represented by Gly-X-Y, the proportion of the sequence represented by Gly-X-Y may be 80% or more and preferably 95% or more of the complete amino acid sequence. In addition, the extracellular matrix molecule may be a polypeptide having an RGD sequence. The RGD sequence is a sequence represented by Arg-Gly-Asp (arginine residue-glycine residue-aspartic acid residue). When the RGD sequence is provided, cell adhesion is further promoted, which makes it more suitable as a scaffolding material. Examples of extracellular matrix molecules including a sequence represented by Gly-X-Y and an RGD sequence include collagen, fibronectin, vitronectin, laminin, and cadherin.

Examples of collagen include fibrous collagen and non-fibrous collagen. Fibrous collagen is collagen including collagen fibers as a main component, and specific examples thereof include type I collagen, type II collagen, and type III collagen. Examples of non-fibrous collagen include type IV collagen.

Examples of proteoglycans include chondroitin sulfate proteoglycan, heparan sulfate proteoglycan, keratan sulfate proteoglycan, and dermatan sulfate proteoglycan, but the present invention is not limited thereto.

The extracellular matrix component may contain at least one selected from the group consisting of collagen, laminin, and fibronectin, and preferably contains collagen. Collagen is preferably fibrous collagen, and more preferably type I collagen. Regarding fibrous collagen, commercially available collagen may be used, and specific examples thereof include porcine skin-derived type I collagen (commercially available from NH Foods Ltd.).

The extracellular matrix component may be an animal-derived extracellular matrix component. Examples of animal species from which an extracellular matrix component is derived include humans, pigs, and cows, but the present invention is not limited thereto. Regarding the extracellular matrix component, a component derived from one kind of animal may be used, or a component derived from a plurality of kinds of animals may be used in combination. The animal species from which an extracellular matrix component is derived may be the same as or different from the origin of cells that are formed into a three-dimensional tissue.

The fragmented extracellular matrix component can be obtained by fragmenting the above extracellular matrix component. "Fragmentation" means that aggregates of extracellular matrix molecules are made smaller in size. Fragmentation may be performed under conditions in which the bond within the extracellular matrix molecule breaks or may be performed under conditions in which the bond within the extracellular matrix molecule does not break. The fragmented extracellular matrix component may include a fibrillated extracellular matrix component which is a component obtained by fibrillating the above extracellular matrix component by applying a physical force. Fibrillation is an aspect of fragmentation, and may be performed, for example, under conditions in which the bond within the extracellular matrix molecule does not break.

A method for fragmenting an extracellular matrix component is not particularly limited. Regarding a method for fibrillating an extracellular matrix component, for example, an extracellular matrix component may be fibrillated by applying a physical force with an ultrasonic homogenizer, a stirring homogenizer, a high pressure homogenizer or the like. When the stirring homogenizer is used, the extracellular matrix component may be homogenized directly or may be homogenized in an aqueous medium such as saline. In addition, a fibrillated extracellular matrix component with a millimeter size or nanometer size can also be obtained by adjusting a homogenizing time, the number of times, and the like. The fibrillated extracellular matrix component can also be obtained according to fibrillating by repeating freezing and melting.

At least a part of the fragmented extracellular matrix component may contain the fibrillated extracellular matrix component. In addition, the fragmented extracellular matrix component may be composed of only the fibrillated extracellular matrix component. That is, the fragmented extracellular matrix component may be a fibrillated extracellular matrix component. The fibrillated extracellular matrix component preferably contains a fibrillated collagen component (fibrillated collagen component). The fibrillated collagen component preferably maintains a triple helix structure derived from collagen. When the fragmented collagen component is dispersed in an aqueous medium, it easily comes into contact with cells in an aqueous medium and can promote formation of the three-dimensional tissue construct.

Examples of the shape of the fragmented extracellular matrix component include a fibrous form. The fibrous form means a shape composed of a filamentous extracellular matrix component or a shape composed of a filamentous extracellular matrix component crosslinked between molecules. At least a part of the fragmented extracellular matrix component may be fibrous. Examples of fibrous extracellular matrix components include fine filaments (fine fibers) formed by aggregating a plurality of filamentous extracellular matrix molecules, filaments formed by additionally aggregating fine fibers, and those obtained by fibrillating these filaments. The fibrous extracellular matrix component can retain the RGD sequence without disruption and can function as a scaffolding material for cell adhesion more effectively.

The average length of the fragmented extracellular matrix component may be 100 nm or more and 400 $\mu$m or less, or 100 nm or more and 200 $\mu$m or less. In one embodiment, in order to facilitate formation of a thick tissue, the average length of the fragmented extracellular matrix component may be 5 $\mu$m or more and 400 $\mu$m or less, 10 $\mu$m or more and 400 $\mu$m or less, 22 $\mu$m or more and 400 $\mu$m or less, or 100 $\mu$m or more and 400 $\mu$m or less. In another embodiment, in order to facilitate stable tissue formation and further improve redispersibility, the average length of the fragmented extracellular matrix component may be 100 $\mu$m or less, 50 $\mu$m or less, 30 $\mu$m or less, 15 $\mu$m or less, 10 $\mu$m or less, 1 $\mu$m or less, or 100 nm or more. The average length of most of the fragmented extracellular matrix component within the entire fragmented extracellular matrix component is preferably within the above numerical value range. Specifically, the average length of 95% of the fragmented extracellular matrix component within the entire fragmented extracellular matrix component is preferably within the above numerical value range. The fragmented extracellular matrix component is preferably a fragmented collagen component having an average length within the above range, and more preferably a fibrillated collagen component having an average length within the above range.

The average diameter of the fragmented extracellular matrix component may be 50 nm to 30 $\mu$m, 4 $\mu$m to 30 $\mu$m, or 5 $\mu$m to 30 $\mu$m. The fragmented extracellular matrix component is preferably a fragmented collagen component having an average diameter within the above range, and more preferably a fibrillated collagen component having an average diameter within the above range.

Since the above ranges of the average length and the average diameter are optimized in consideration of tissue formation, it is desirable that the average length or the average diameter be within the above range at a step of suspending the fragmented extracellular matrix component again in an aqueous medium to form a tissue after a drying process to be described below.

The average length and average diameter of the fragmented extracellular matrix components can be determined by measuring each fragmented extracellular matrix component under an optical microscope and performing image analysis. In this specification, the "average length" is an average value of the lengths of the measured samples in the longitudinal direction, and the "average diameter" is an average value of lengths of the measured samples in a direction orthogonal to the longitudinal direction.

At least a part of the fragmented extracellular matrix component may be crosslinked intermolecularly or intramolecularly. The fragmented extracellular matrix component may be crosslinked within molecules constituting the fragmented extracellular matrix component or may be crosslinked between molecules constituting the fragmented extracellular matrix component.

Examples of a crosslinking method include a physical cross-linking method of applying heat, ultraviolet rays, radiation and the like, and a chemical crosslinking method using a crosslinking agent, an enzymatic reaction and the like, but the method is not particularly limited. Crosslinking (physical cross-linking and chemical crosslinking) may be crosslinking via a covalent bond.

When the extracellular matrix component contains a collagen component, crosslinks may be formed between collagen molecules (triple helix structure), or may be formed between collagen fine fibers formed by collagen molecules. The crosslinking may be crosslinking by heat (thermal crosslinking). For example, thermal crosslinking can be performed by performing a heat treatment under a reduced pressure using a vacuum pump. When the collagen component is thermally crosslinked, the extracellular matrix component may be crosslinked when amino groups of collagen molecules form a peptide bond (-NH-CO-) with carboxyl groups of the same or other collagen molecules.

The extracellular matrix component can also be crosslinked by using a crosslinking agent. For example, the crosslinking agent may be an agent that can crosslink carboxyl groups and amino groups or an agent that can crosslink amino groups with each other. Regarding the crosslinking agent, for example, aldehyde-based, carbodiimide-based, epoxide-based and imidazole-based crosslinking agents are preferable in consideration of economic efficiency, safety and operability, and specific examples thereof include water-soluble carbodiimides such as glutaraldehyde, 1-ethyl-3-(3-dimethylaminopropyl)carbodiimide/hydrochloride, and 1-cyclohexyl-3-(2-morpholinyl-4-ethyl)carbodiimide/sulfonate.

Quantification of the degree of crosslinking can be appropriately selected depending on the type of extracellular matrix component, a crosslinking method, and the like. The degree of crosslinking may be 1% or more, 2% or more, 4% or more, 8% or more, or 12% or more, or may be 30% or less, 20% or less, or 15% or less. When the degree of crosslinking is within the above range, extracellular matrix molecules can be appropriately dispersed, and the redispersibility after dry storage is favorable.

When the amino group in the extracellular matrix component is used for crosslinking, the degree of crosslinking can be quantified based on the TNBS method described in Non-Patent Literature 2 and the like. The degree of crosslinking by the TNBS method is preferably within the above range. The degree of crosslinking by the TNBS method is a proportion of amino groups used for crosslinking among amino groups of the extracellular matrix.

The degree of crosslinking may be calculated by quantifying carboxyl groups. For example, an extracellular matrix component that is insoluble in water may be quantified by a TBO (toluidine blue O) method. The degree of crosslinking by the TBO method may be within the above range.

The content of the fragmented extracellular matrix component (fragmented extracellular matrix component) in the extracellular matrix-containing composition based on a total amount of the extracellular matrix-containing composition may be 1 mass% or more, 3 mass% or more, 10 mass% or more, 20 mass% or more, 30 mass% or more, 40 mass% or more, 50 mass% or more, 60 mass% or more, 70 mass% or more, 80 mass% or more, 90 mass% or more, 95 mass% or more, or 98 mass% or more, or may be 99 mass% or less, 95 mass% or less, or 90 mass% or less.

The extracellular matrix-containing composition according to the present embodiment contains a compound bound or adsorbed to a fragmented extracellular matrix component. The compound may be a biomolecule as long as it can bind or adsorb to the fragmented extracellular matrix component contained in the extracellular matrix-containing composition. In this specification, being able to bind or adsorb is synonymous with being able to coat a fibrillated extracellular matrix component with a compound. In addition, when the fibrillated extracellular matrix component is coated, the entire surface may be coated or a part thereof may be coated. The compound can be appropriately selected depending on the type of the fragmented extracellular matrix component. The compound may be, for example, an extracellular matrix component containing extracellular matrix molecules that are the same species as or different species from those of the fragmented extracellular matrix component. Specific examples of compounds include chondroitin sulfate, heparan sulfate, fibronectin, heparin, proteoglycan, hyaluronic acid, heparan sulfate proteoglycan, chondroitin sulfate proteoglycan, laminin, entactin, tenascin, elastin, and fibrillin, but the present invention is not limited thereto. For example, the compound may be at least one selected from the group consisting of heparan sulfate, chondroitin sulfate, and fibronectin. When the fragmented extracellular matrix component is a fragmented collagen component, the compound may be chondroitin sulfate, heparan sulfate, heparin, or fibronectin. The fragmented extracellular matrix component to which the compound is bound or adsorbed has a function that the fragmented extracellular matrix component alone does not have or an enhanced function of the fragmented extracellular matrix component alone. For example, chondroitin sulfate improves the adhesive strength between cells. Heparin facilitates cell growth. Fibronectin improves the adhesiveness of cells to the fragmented extracellular matrix component.

The content of the compound with respect to 100 parts by mass of the fragmented extracellular matrix component may be 0.5 parts by mass or more and 10 parts by mass or less, 1.0 part by mass or more and 8.0 parts by mass or less, or 1.5 parts by mass or more and 4.0 parts by mass or less. The content of the compound can be calculated, for example, based on an adsorption rate or the like described in examples to be described below.

The extracellular matrix-containing composition may be composed of only a fragmented extracellular matrix component and the above compound, and may contain a fragmented extracellular matrix component and a component other than the above compound (other component).

The form of the extracellular matrix-containing composition may be a solid or powder form because in this case it is easy to weigh out. The extracellular matrix-containing composition does not have to contain water. Water in the extracellular matrix-containing composition can be removed by, for example, a freeze-drying method. "Does not contain water" does not mean that it does not contain any water molecules, but means not containing water to a common sense extent attained according to a drying method such as a freeze-drying method.

The extracellular matrix-containing composition according to one embodiment can be dispersed in an aqueous medium. The "aqueous medium" is a liquid containing water as an essential component. The aqueous medium is not particularly limited as long as the extracellular matrix component can be stably present. Examples of aqueous mediums include liquid mediums, for example, saline such as phosphate buffered saline (PBS), a Dulbecco's Modified Eagle culture medium (DMEM), and a vascular endothelial cell growth culture medium (EGM2), but the present invention is not limited thereto.

Dispersibility in an aqueous medium is determined by, for example, the following method. That is, when 50 mg of the extracellular matrix-containing composition is added to 5 mL of ultrapure water and suspended, if the extracellular matrix-containing composition is dispersed in ultrapure water (if no aggregation or the like occurs), it can be determined that it can be dispersed in the aqueous medium. The temperature at which the extracellular matrix-containing composition is dispersed in ultrapure water may be a temperature equal to or lower than the culture temperature (for example, 37°C) or may be room temperature. The dispersed state is a state in which aggregations, precipitations and the like are not visually observed. Here, dispersibility can be determined by, for example, measuring the absorbance.

The pH of the aqueous medium is preferably in a range that does not adversely affect the growth of cells and formation of cell aggregates. The pH of the aqueous medium may be, for example, 7.0 or more or 8.0 or less, in order to reduce the load on cells when injected into the cells. Specifically, the pH of the aqueous medium may be 7.0, 7.1, 7.2, 7.3, 7.4, 7.5, 7.6, 7.7, 7.8, 7.9, or 8.0. The aqueous medium is preferably a medium having a buffer capacity in the above pH range and more preferably a liquid medium. The liquid medium is not particularly limited, and an appropriate culture medium can be selected according to the type of cells to be cultured. Examples of the culture medium include an Eagle's MEM culture medium, DMEM, a Modified Eagle culture medium (MEM), a Minimum Essential culture medium, RPMI, and a GlutaMax culture medium. The culture medium may be a culture medium to which serum is added or a serum-free culture medium. In addition, the liquid medium may be a mixed culture medium in which two or more types of culture mediums are mixed.

<Method for producing extracellular matrix-containing composition>

A method for producing an extracellular matrix-containing composition according to the present embodiment includes a process (contact process) of bringing a fragmented extracellular matrix component into contact with a compound that can be bound or adsorbed to the fragmented extracellular matrix component. Examples of the contact process include a method such as mixing an aqueous medium containing a fragmented extracellular matrix component and an aqueous medium containing a compound, and adding a compound to an aqueous medium containing a fragmented extracellular matrix component, but the present invention is not limited thereto. In addition, the contact process may include a process of performing incubating for a certain time after the fragmented extracellular matrix component is brought into contact with the compound.

The fragmented extracellular matrix component can be obtained by the above method. The fragmented extracellular matrix component may be obtained by fragmenting the extracellular matrix component in an aqueous medium. That is, the production method according to the present embodiment may include a process of fragmenting the extracellular matrix component in an aqueous medium (fragmentation process) before the contact process. The aqueous medium may be the same as the above aqueous medium. The fragmentation process may be a process of fibrillating the extracellular matrix component in an aqueous medium before the contact process.

The fragmented extracellular matrix component may be those exemplified above, and may contain a fragmented collagen component or may contain a fibrillated collagen component. As the compound that can be bound or adsorbed to the fragmented extracellular matrix component, the above compound can be used.

The production method according to the present embodiment may include a process of heating the extracellular matrix component before the fragmentation process and crosslinking at least a part of the extracellular matrix component, or may include a process of heating the extracellular matrix component and crosslinking at least a part of the extracellular matrix component after the fragmentation process and before the contact process.

In the crosslinking process, the temperature (heating temperature) and the time (heating time) when the extracellular matrix component is heated can be appropriately determined. The heating temperature may be, for example, 100°C or higher or 200°C or lower. The heating temperature may be, specifically, for example, 100°C, 110°C, 120°C, 130°C, 140°C, 150°C, 160°C, 170°C, 180°C, 190°C, or 200°C. The heating time (time during which the heating temperature is maintained) can be appropriately set depending on the heating temperature. When heating is performed at, for example, 100°C to 200°C, the heating time may be 6 hours or longer and 72 hours or shorter, and more preferably 24 hours or longer and 48 hours or shorter. In the crosslinking process, heating may be performed in the absence of a solvent or heating may be performed under a reduced pressure condition.

The production method according to the present embodiment may include a drying process of drying the fragmented extracellular matrix component after the fragmentation process.

In the drying process, the fragmented extracellular matrix component is dried. Drying may be performed by, for example, a freeze-drying method. When the drying process is performed after the fragmentation process, the aqueous medium is removed from the liquid containing the fragmented extracellular matrix component and the aqueous medium. Removal of the aqueous medium does not mean that no water is attached to the fragmented extracellular matrix component, but means that there is not enough water attached to a common sense extent attained according to the above general drying method.

The extracellular matrix-containing composition can be suitably used as a scaffolding material for forming a three-dimensional tissue construct. Therefore, the extracellular matrix-containing composition is suitably used for three-dimensional tissue construct formation applications.

<Three-dimensional tissue construct forming agent>

Since the extracellular matrix-containing composition is suitable as a scaffolding material or the like for forming a three-dimensional tissue construct, in one embodiment of the present invention, a three-dimensional tissue construct forming agent containing the above extracellular matrix-containing composition is provided.

Since the three-dimensional tissue construct forming agent according to the present embodiment contains the above fibrillated extracellular matrix component, a thicker three-dimensional tissue construct can be formed.

A three-dimensional tissue construct forming agent may be in a powder form during storage, and is preferably in a dispersed liquid form dispersed in an aqueous medium in a step of forming a three-dimensional tissue construct.

<Three-dimensional tissue construct>

The three-dimensional tissue construct according to the present embodiment contains the above extracellular matrix-containing composition and cells. At least some of cells may be in contact with the fibrillated extracellular matrix component in the extracellular matrix-containing composition. As one aspect of contact, they may be adhered. The "three-dimensional tissue construct" is an aggregate of cells in which cells are three-dimensionally arranged via an extracellular matrix component, and is an aggregate artificially produced by cell culture. The shape of the three-dimensional tissue construct is not particularly limited, and examples thereof include a sheet shape, a spherical shape, an ellipsoidal shape, and a rectangular parallelepiped shape. Here, the living tissue includes blood vessels, sweat glands, lymphatic vessels, sebaceous glands, and the like, and has a configuration that is more complicated than that of the three-dimensional tissue construct. Therefore, the three-dimensional tissue construct and the living tissue can be easily distinguished.

The cells are not particularly limited, and may be, for example, cells derived from animals such as humans, monkeys, dogs, cats, rabbits, pigs, cows, mice, and rats. The parts of cells derived are not particularly limited, and may be somatic cells derived from bones, muscles, internal organs, nerves, brain, bones, skin, blood, and the like or may be germ cells. In addition, the cells may be induced pluripotent stem cells (iPS cells), embryonic stem cells (ES cells) or may be cultured cells such as primary cultured cells, subcultured cells and cell line cells. Specific examples of cells include nerve cells, dendritic cells, immune cells, vascular endothelial cells (for example, human umbilical vein endothelial cells (HUVEC)), vascular pericytes, lymphatic endothelial cells, fibroblasts, colon cancer cells (for example, human colon cancer cells (HT29)), cancer cells such as liver cancer cells, epithelial cells (for example, human gingival epithelial cells), keratinocytes, cardiomyocytes (for example, human iPS cell-derived cardiomyocytes (iPS-CM)), liver cells, pancreatic islet cells, tissue stem cells, and smooth muscle cells (for example, aortic smooth muscle cells (Aorta-SMC)), but the present invention is not limited thereto. The cells may include one or more types of cells selected from the group consisting of vascular endothelial cells, cancer cells, cardiomyocytes, smooth muscle cells, and epithelial cells. In addition, the cells may include vascular endothelial cells, vascular pericytes and astrocyte. The cells may be used alone or a plurality of types of cells may be used in combination.

The cells preferably include extracellular matrix secreting cells that secrete extracellular matrix molecules. Examples of extracellular matrix secreting cells include collagen secreting cells that secrete collagen such as fibrous collagen. Examples of collagen secreting cells include mesenchymal cells such as fibroblasts, chondrocytes, and osteoblasts, and fibroblasts are preferable. Examples of preferable fibroblasts include normal human dermal fibroblasts (NHDF), normal human cardiac fibroblasts (NHCF) and human gingival fibroblasts (HGF).

When the three-dimensional tissue construct contains extracellular matrix secreting cells as cells, the three-dimensional tissue construct may contain an endogenous extracellular matrix. The "endogenous extracellular matrix" is an extracellular matrix produced by extracellular matrix producing cells constituting a three-dimensional tissue construct.

When the three-dimensional tissue construct contains collagen secreting cells as cells, the three-dimensional tissue construct may contain endogenous collagen. The "endogenous collagen" is collagen produced by collagen producing cells constituting a three-dimensional tissue construct. The endogenous collagen may be fibrous collagen or non-fibrous collagen.

When the three-dimensional tissue construct contains extracellular matrix secreting cells as cells, the three-dimensional tissue construct may contain cells containing extracellular matrix secreting cells, an extracellular matrix-containing composition, and an endogenous extracellular matrix component. In this case, at least some of cells containing extracellular matrix secreting cells may be in contact with the fragmented extracellular matrix component and/or the endogenous extracellular matrix component. A conventional three-dimensional tissue construct has a low concentration of the extracellular matrix (collagen, etc.) and has a high cell density. Therefore, there are problems of the three-dimensional tissue construct shrinking due to a traction force of cells during culture or after culture and the three-dimensional tissue construct being easily decomposed by the enzyme produced by cells during culture or after culture. The three-dimensional tissue construct according to one embodiment has a higher concentration of the extracellular matrix (collagen, etc.) than the conventional one, is less likely to shrink and is stable.

The three-dimensional tissue construct may include extracellular matrix secreting cells as cells and cells other than the extracellular matrix secreting cells. Examples of cells other than the extracellular matrix producing cells include vascular endothelial cells (for example, human umbilical vein endothelial cells (HUVEC)), colon cancer cells (for example, human colon cancer cells (HT29)), cancer cells such as liver cancer cells, cardiomyocytes (for example, human iPS cell-derived cardiomyocytes (iPS-CM)), epithelial cells (for example, human gingival epithelial cells), keratinocytes, lymphatic endothelial cells, nerve cells, liver cells, tissue stem cells, embryonic stem cells, induced pluripotent stem cells, adherent cells (for example, immune cells), and smooth muscle cells (for example, aortic smooth muscle cells (Aorta-SMC)). The cells constituting the above three-dimensional tissue construct preferably further include one or more types of cells selected from the group consisting of vascular endothelial cells, cancer cells, cardiomyocytes, smooth muscle cells, and epithelial cells.

The total content of the fragmented extracellular matrix component and the compound in the three-dimensional tissue construct based on a total mass of the fragmented extracellular matrix component, the compound and cells may be 0.01 mass% or more and 90 mass% or less, 10 mass% or more and 90 mass% or less, 10 mass% or more and 80 mass% or less, 10 mass% or more and 70 mass% or less, 10 mass% or more and 60 mass% or less, 1 mass% or more and 50 mass% or less, 10 mass% or more and 50 mass% or less, 10 mass% or more and 30 mass% or less, or 20 to 30 mass%. The total content of the fragmented extracellular matrix component and the compound can be measured by, for example, general MS imaging.

When the three-dimensional tissue construct contains collagen, the content of collagen in the three-dimensional tissue construct based on a total mass of the fragmented extracellular matrix component, the compound and cells may be 0.01 to 90 mass%, and is preferably 0.33 to 90 mass%, preferably 5 to 90 mass%, preferably 10 to 90 mass%, preferably 10 to 80 mass%, preferably 10 to 70 mass%, preferably 10 to 60 mass%, preferably 1 to 50 mass%, preferably 10 to 50 mass%, more preferably 10 to 30 mass%, and more preferably 20 to 30 mass%.

Here, "collagen in the three-dimensional tissue construct" is collagen constituting the three-dimensional tissue construct and may be endogenous collagen or collagen derived from a fragmented collagen component (exogenous collagen). That is, when the three-dimensional tissue construct contains an endogenous collagen component and a fragmented collagen component, the content of collagen constituting the three-dimensional tissue construct is a total concentration of the endogenous collagen component and the fragmented collagen component. The content of collagen can be calculated from the volume of the obtained three-dimensional tissue construct and the mass of the decellularized three-dimensional tissue construct.

In addition, examples of a method for quantifying the amount of collagen in the three-dimensional tissue construct include a method for quantifying hydroxyproline as will be described below. A sample is prepared by mixing hydrochloric acid (HCl) with a dissolved solution in which a three-dimensional tissue construct is dissolved, incubating the mixture at a high temperature for a predetermined time, and then returning the temperature to room temperature, and diluting the centrifuged supernatant to a predetermined concentration. A hydroxyproline standard solution is treated in the same manner as in the sample, and then diluted stepwise to prepare a standard. The sample and the standard are subjected to a predetermined treatment with a hydroxyproline assay buffer and a detection reagent, and the absorbance at 570 nm is measured. The amount of collagen is calculated by comparing the absorbance of the sample with the standard. Here, a dissolved solution in which the three-dimensional tissue construct is directly suspended and dissolved in hydrochloric acid with a high concentration is centrifuged to recover the supernatant and may be used for collagen quantification. In addition, the three-dimensional tissue construct to be dissolved may be in a state in which it is recovered from the culture solution, or may be dried after recovering, or to be dissolved in a state in which the liquid component has been removed. However, when collagen is quantified by dissolving the three-dimensional tissue construct in a state in which it is recovered from the culture solution, since it is expected that the measured value of the weight of the three-dimensional tissue construct will vary due to the culture medium component adsorbed by the three-dimensional tissue construct and the remaining influence of the culture medium due to the problem of the experimental technique, in order to stably measure the weight of the tissue construct and the amount of collagen per unit weight, the weight after drying is preferable as a reference.

More specifically, examples of a method for quantifying an amount of collagen include the following method.

(Preparation of sample)

The entire amount of the freeze-dried three-dimensional tissue construct is mixed with 6 mol/l HCl, and the mixture is incubated in a heat block at 95°C for 20 hours or longer, and the temperature is then returned to room temperature. Centrifuging is performed at 13,000 g for 10 minutes and the supernatant of the sample solution is then recovered. A sample is prepared by appropriately diluting with 6 mol/l HCl so that the result falls within the range of the calibration curve in the measurement described below, and then diluting 200 $\mu$L with 100 $\mu$L of ultrapure water. 35 $\mu$L of the sample is used.

(Preparation of standard)

125 $\mu$L of a standard solution (1,200 $\mu$g/mL in acetic acid) and 125 $\mu$L of 12 mol/l HCl are added to a screw cap tube and mixed, and incubated in a heat block at 95°C for 20 hours, and the temperature is then returned to room temperature. Centrifuging is performed at 13,000 g for 10 minutes and the supernatant is then diluted with ultrapure water to produce 300 $\mu$g/mL of S1, and S1 is diluted stepwise to produce S2 (200 $\mu$g/mL), S3 (100 $\mu$g/mL), S4 (50 $\mu$g/mL), S5 (25 $\mu$g/mL), S6 (12.5 $\mu$g/mL), and S7 (6.25 $\mu$g/mL). S8 (0 $\mu$g/mL) containing only 90 $\mu$L of 4 mol/l HCl is also prepared.

(Assay)

35 $\mu$L of each of the standard and the sample is added to a plate (bundled in QuickZyme Total Collagen Assay kit, commercially available from QuickZyme Biosciences). 75 $\mu$L of an assay buffer (bundled in the above kit) is added to each well. The plate is closed with a seal, and incubation is performed at room temperature for 20 minutes while shaking. The seal is removed and 75 $\mu$L of a detection reagent (reagent A:B=30 $\mu$L:45 $\mu$L, bundled in the above kit) is added to each well. The plate is closed with a seal, and the solution is mixed with shaking, and incubated at 60°C for 60 minutes. Sufficient cooling is performed on ice, the seal is removed, and the absorbance at 570 nm is measured. The amount of collagen is calculated by comparing the absorbance of the sample with the standard.

In addition, collagen in the three-dimensional tissue construct may be determined by its area ratio or volume ratio. "Determined by the area ratio or volume ratio" means that, for example, collagen in the three-dimensional tissue construct is made distinguishable from other tissue structures by a known staining method (for example, immunostaining using anti-collagen antibodies or Masson's trichrome staining) or the like, and the ratio of the area of collagen in the entire three-dimensional tissue construct is then calculated by observation with the naked eye and using various microscopes, image analysis software, and the like. When determined according to the area ratio, there is no limitation on which cross section or surface in the three-dimensional tissue construct is used for determination of the area ratio, but for example, when the three-dimensional tissue construct is a spherical body or the like, it may be determined by a cross-sectional view that passes through substantially a central part thereof.

For example, when collagen in the three-dimensional tissue construct is determined by the area ratio, the ratio of the area based on a total area of the three-dimensional tissue construct is 0.01 to 99%, preferably 1 to 99%, preferably 5 to 90%, preferably 7 to 90%, preferably 20 to 90%, and more preferably 50 to 90%. "Collagen in the three-dimensional tissue construct" is as described above. When the three-dimensional tissue construct contains exogenous collagen, the ratio of the area of collagen constituting the three-dimensional tissue construct is the ratio of the total area of endogenous collagen and exogenous collagen. For example, the obtained three-dimensional tissue construct is stained with Masson's trichrome, and the ratio of the area of collagen can be calculated as the ratio of the area of collagen stained with blue with respect to the total area of the cross section that passes through substantially a central part of the three-dimensional tissue construct.

The residual ratio of the three-dimensional tissue construct subjected to a trypsin treatment with a trypsin concentration of 0.25%, a temperature of 37°C, a pH of 7.4, and a reaction time of 15 minutes is preferably 70% or more, more preferably 80% or more, and still more preferably 90% or more. Such a three-dimensional tissue construct is stable because it is unlikely to be decomposed by an enzyme during culture or after culture. The residual ratio can be calculated from, for example, the mass of the three-dimensional tissue construct before and after the trypsin treatment.

The residual ratio of the three-dimensional tissue construct subjected to a collagenase treatment with a collagenase concentration of 0.25%, a temperature of 37°C, a pH of 7.4, and a reaction time of 15 minutes may be 70% or more, and is more preferably 80% or more, and still more preferably 90% or more. Such a three-dimensional tissue construct is stable because it is unlikely to be decomposed by an enzyme during culture or after culture.

The thickness of the three-dimensional tissue construct is preferably 10 $\mu$m or more, more preferably 100 $\mu$m or more, and still more preferably 1,000 $\mu$m or more. Such a three-dimensional tissue construct is a structure closer to that of a living tissue, and is suitable as a substitute for laboratory animals and a transplant material. The upper limit of the thickness of the three-dimensional tissue construct is not particularly limited, and may be, for example, 10 mm or less, 3 mm or less, 2 mm or less, 1.5 mm or less, or 1 mm or less.

Here, the "thickness of the three-dimensional tissue construct" is the distance between both ends in a direction perpendicular to the main surface when the three-dimensional tissue construct has a sheet shape or a rectangular parallelepiped shape. When the main surface is uneven, the thickness is the distance at the thinnest part of the main surface.

In addition, when the three-dimensional tissue construct has a spherical shape, the thickness is its diameter. In addition, when the three-dimensional tissue construct has an ellipsoidal shape, the thickness is its minor axis. When the three-dimensional tissue construct has a substantially spherical shape or a substantially ellipsoidal shape and the surface is uneven, the thickness is the shortest distance between two points at which a straight line passing through the center of gravity of the three-dimensional tissue construct and the surface intersect.

<Method for producing three-dimensional tissue construct>

The method for producing a three-dimensional tissue construct according to the present embodiment includes a first process in which, in an aqueous medium, the above extracellular matrix-containing composition is brought into contact with cells and a second process in which the cells in contact with the above extracellular matrix-containing composition are cultured. An important point in the method for producing a three-dimensional tissue construct according to the present embodiment is that, before the fragmented extracellular matrix component is brought into contact with cells (that is, the first process), the fragmented extracellular matrix component comes into contact with a compound, and the compound binds or adsorbs to the fragmented extracellular matrix component. This makes it possible to impart or enhance useful functions of the fragmented extracellular matrix component compared with when a compound is simply added to a cell culture solution and cells are cultured. In addition, since the compound is bound or adsorbed to the fragmented extracellular matrix component, there is also an effect of reducing the amount of the compound used.

In the method for producing a three-dimensional tissue construct, the cells are preferably cells containing collagen producing cells. When cells containing collagen secreting cells are used, a three-dimensional tissue construct which is more stable and in which the cells are uniformly distributed can be obtained. The details of the mechanism by which such a three-dimensional tissue construct is obtained are unknown, but are inferred as follows.

In a conventional method for producing a three-dimensional tissue construct using a scaffold, since target cells are injected into a scaffold prepared in advance, it is difficult to uniformly distribute cells even inside the scaffold. When the cells are cells containing extracellular matrix producing cells (collagen producing cells, etc.), first, the cells adhere to the extracellular matrix-containing composition. Then, the cells themselves produce proteins constituting the extracellular matrix component (for example, collagen such as fibrous collagen). When the produced proteins are adhered to the extracellular matrix-containing composition, they function as a crosslinking agent between the extracellular matrix-containing compositions, and in an environment in which cells are uniformly present, structuring of the proteins and the like constituting the extracellular matrix component progresses. As a result, a three-dimensional tissue construct which is more stable and in which the cells are uniformly distributed can be obtained. However, the above inference does not limit the present invention.

In addition, in the production methods described in Patent Literature 1 to 3, the number of processes for producing the three-dimensional tissue construct is large and an operation time of about 1 hour is required. According to the production method according to the present embodiment, it is possible to produce a three-dimensional tissue construct in a short operation time. In addition, according to the production method according to the present embodiment, it is possible to easily produce a three-dimensional tissue construct. In the production method described in Patent Literature 2, in order to produce a three-dimensional tissue construct with a thickness of about 1 mm, at least $10^6$ cells are required. According to the production method according to the present embodiment, it is possible to produce a large-sized three-dimensional tissue construct with a thickness of 1 mm or more with a relatively a small number of cells.

In the first process, in the aqueous medium, the extracellular matrix-containing composition is brought into contact with cells. In the aqueous medium, a method for bringing the extracellular matrix-containing composition into contact with cells is not particularly limited. Examples thereof include a method in which an extracellular matrix-containing composition is added to a culture solution containing cells, a method in which an aqueous medium and cells are added to an extracellular matrix-containing composition, and a method in which an extracellular matrix-containing composition and cells are added to an aqueous medium prepared in advance.

In the first process, cells containing collagen producing cells and cells other than collagen producing cells may be used. Regarding collagen producing cells, and cells other than collagen producing cells, the above cells can be used. When a three-dimensional tissue construct is produced using collagen producing cells and cells other than collagen producing cells together, it is possible to produce various model tissues. For example, when NHCF and HUVEC are used, it is possible to obtain a three-dimensional tissue construct having capillary vessels therein. When NHCF and colon cancer cells are used, it is possible to obtain a model tissue for colon cancer. In addition, when NHCF and iPS-CM are used, it is possible to obtain a model tissue of myocardium showing synchronous pulsation.

The concentration of the extracellular matrix-containing composition in the first process can be appropriately determined according to the shape and thickness of a desired three-dimensional tissue construct, the size of a culture container, and the like. For example, the concentration of the extracellular matrix-containing composition in the aqueous medium in the first process may be 0.1 to 90 mass% or 1 to 30 mass%.

The amount of the extracellular matrix-containing composition in the first process may be 0.1 to 100 mg or 1 to 50 mg with respect to $1 \times 10^5$ cells.

In the first process, the mass ratio between the extracellular matrix-containing composition and the cells (extracellular matrix-containing composition/cells) is preferably 1/1 to 1,000/1, more preferably 9/1 to 900/1, and still more preferably 10/1 to 500/1.

When collagen producing cells and other cells are used together, the ratio of the number of collagen producing cells in the first process with respect to the proportion of other cells (ratio of collagen producing cells/other cells in the first process) may be 9/1 to 99/1, 50/50 to 80/20, 20/80 to 50/50, or 10/90 to 50/50.

After the first process and before the second process, a process in which the extracellular matrix-containing composition and cells are precipitated together in an aqueous medium may be additionally provided. When such a process is performed, the distribution of the extracellular matrix-containing composition and cells in the three-dimensional tissue construct becomes more uniform. A specific method is not particularly limited, and examples thereof include a method for centrifuging a culture solution containing an extracellular matrix-containing composition and cells.

The first process may be performed after a layer of cells is formed in the aqueous medium. That is, the first process may be performed by forming a layer of cells in the aqueous medium and then bringing the extracellular matrix-containing composition into contact therewith. When the cell layer is formed before it is brought into contact with the extracellular matrix-containing composition, it is possible to produce a three-dimensional tissue construct with a lower layer part having a high cell density. In addition, when a layer of cells containing collagen producing cells is formed before it is brought into contact with the extracellular matrix-containing composition, it is possible to produce a three-dimensional tissue construct with a lower layer part of cells containing collagen producing cells with a high cell density. Depending on the type of cells used (for example, aortic smooth muscle cells), it is possible to produce a tissue closer to a living body according to this method.

After the second process, as a third process, a process in which cells are brought into contact therewith and the cells are cultured may be additionally provided. The cells may be the same species as or different species from the cells used in the first process. For example, when the cells used in the first process contain cells other than the collagen producing cells, the cells used in the third process may contain collagen producing cells. In addition, for example, when the cells used in the first process contain collagen producing cells, the cells used in the third process may contain cells other than the collagen producing cells. Both the cells used in the first process and the cells used in the third process may contain collagen producing cells, and both the cells used in the first process and the cells used in the third process may contain cells other than the collagen producing cells. In the third process, it is possible to produce a three-dimensional tissue construct having a two-layer structure. For example, when aortic smooth muscle cells and vascular endothelial cells are used, and when normal human dermal fibroblasts and human epidermal keratinocytes are used, it is possible to produce a tissue closer to a living body according to this method. In addition, for example, when human gingival fibroblasts and gingival epithelial cells are used, it is possible to produce a three-dimensional tissue construct having a two-layer structure without tissue shrinkage and tissue breaking according to this method.

A method for culturing cells with which the extracellular matrix-containing composition is brought into contact is not particularly limited, and an appropriate culture method can be performed according to the type of cells to be cultured. For example, the culture temperature may be 20°C to 40°C, or 30°C to 37°C. The pH of the culture medium may be 6 to 8, or 7.2 to 7.4. The culture time may be 1 day to 2 weeks, or 1 week to 2 weeks.

The culture medium is not particularly limited, and an appropriate culture medium can be selected according to the type of cells to be cultured. Examples of culture mediums include an Eagle's MEM culture medium, a DMEM, a Modified Eagle culture medium (MEM), a Minimum Essential culture medium, an RPMI, and a GlutaMax culture medium. The culture medium may be a culture medium to which serum is added, or a serum-free culture medium. In addition, the liquid medium may be a mixed culture medium in which two or more types of culture mediums are mixed.

The cell density in the culture medium in the second process can be appropriately determined according to the shape and thickness of a desired three-dimensional tissue construct, the size of a culture container, and the like. For example, the cell density in the culture medium in the second process may be 1 to $10^8$ cells/ml, or $10^3$ to $10^7$ cells/ml. In addition, the cell density in the culture medium in the second process may be the same as the cell density in the aqueous medium in the first process.

The shrinkage rate of the three-dimensional tissue construct during culturing is preferably 20% or less, more preferably 15% or less, and still more preferably 10% or less. The shrinkage rate can be calculated by, for example, the following formula. In the formula, L1 indicates a length of the longest part of the three-dimensional tissue construct on the first day after culture, and L3 indicates a length of the corresponding part of the three-dimensional tissue construct on the third day after culture.

$$\text{Shrinkage rate (\%)}=\{(L1\text{-}L3)/L1\}\times 100$$

According to the above production method, for example, it is possible to produce a three-dimensional tissue construct containing cells and an extracellular matrix component in which the content of collagen is 10 mass% to 90 mass% based on the three-dimensional tissue construct.

**Examples**

Hereinafter, the present invention will be described in detail with reference to examples, but the present invention is not limited thereto.

<Test Example 1: Production of fibrillated collagen component>

A freeze-dried type I collagen derived from porcine skin (commercially available from NH Foods Ltd.) was suspended in ultrapure water so that the content of collagen was 1 mass%. The suspension was homogenized using an ultrasonic homogenizer by repeating a cycle for 20 seconds 10 times under a condition of 4°C. The obtained solution was filtered through a filter having a pore size of 35 $\mu$m to obtain a fibrillated collagen component (sCMF).
The average length (length) of sCMF was 14.8±8.2 $\mu$m (N=20).

<Test Example 2: Adsorption of fibrillated collagen component (sCMF) and biomolecule>

The following Compounds 1 to 4 were prepared.

Compound 1: Fluorescein amine-labeled sodium hyaluronate (PG RESEARCH, FAHA-H1)
Compound 2: Fluorescein amine-labeled sodium heparan sulfate (PG RESEARCH, FAHS-P1)
Compound 3: Fluorescein amine-labeled chondroitin sulfate A sodium (PG RESEARCH, FACS-A1)
Compound 4: Rhodamine-labeled fibronectin (Cytoskelton, Inc., Rhodamine fibronectin)

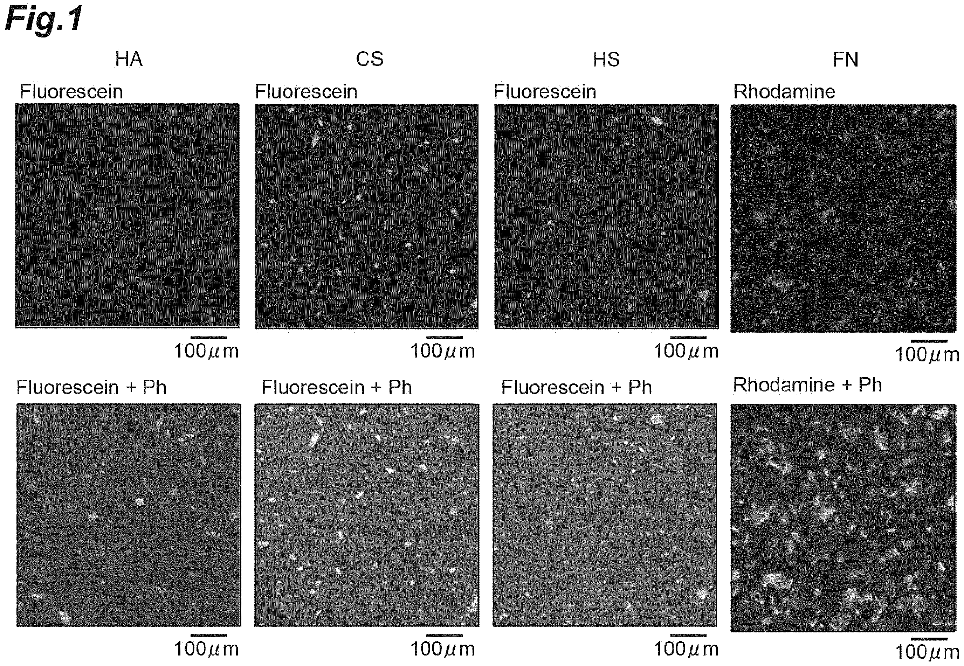

Compounds 1 to 4 were diluted with phosphate buffered saline (PBS) to prepare a solution containing any of Compounds 1 to 4 at a concentration of 0.04 mg/ml.
1 mL of the prepared solution was added to 1 mg of sCMF to obtain a mixed solution. The mixed solution was stirred at room temperature (15 to 25°C) under a condition of 20 rpm for 60 minutes. After stirring was completed, the mixed solution was centrifuged, the supernatant was removed, and washing with PBS was performed to prepare a test sample. FIG. 1 shows the observation results of the test solution prepared using any of Compounds 1 to 4.
In the test samples prepared using fluorescein amine-labeled sodium heparan sulfate, fluorescein amine-labeled chondroitin sulfate A sodium, and rhodamine-labeled fibronectin, binding or adsorption between sCMF and the compound was confirmed according to phase difference (Ph) observation and fluorescence observation. On the other hand, in the test sample prepared using fluorescein amine-labeled sodium hyaluronate, no binding or adsorption between sCMF and the compound was confirmed.

<Test Example 3: Evaluation of adsorption rate of biomolecule>

Solutions in which each of Compound 1 (HA), Compound 2 (CS) and Compound 3 (HS) was diluted with PBS so that the concentration was 0.04 mg/ml (referred to as Solutions 1 to 3) were prepared.
Solutions 1 to 3 were diluted with PBS, and a solution for creating a calibration curve was prepared so that the concentration of Compounds 1 to 3 was 0.1 mg/ml, 0.05 mg/ml, 0.025 mg/ml, or 0.0125 mg/ml. The fluorescence intensity of the solution for creating a calibration curve was measured using a spectrofluorometer (FP-8500 commercially available from JASCO Corporation) to create a calibration curve.

1 mg of sCMF was put into a 1.5 ml sample tube (WATSON, 131-7155C), 1 ml of any one of Solutions 1 to 3 was added thereto, and the mixture was stirred using ROTATOR (TAITEC, RT-50) at room temperature for 1 hour at a speed of 20 rpm. The obtained mixed solution was centrifuged using a centrifugal separator (Eppendorf, minispin) under conditions of 3,500 rpm for 5 minutes, and the fluorescence intensity of 200 μL of the supernatant was measured using a spectrofluorometer. The obtained result was applied to the calibration curve, and the concentration of the fluorescent reagent in the supernatant was calculated. When this value was set as X, the adsorption rate was calculated by the formula of (0.04-X)/0.04×100. Table 1 shows the measurement results of the adsorption rate.

[Table 1]

|  | Compound 1 | Compound 2 | Compound 3 |
|---|---|---|---|
|  | HA | CS | HS |
| Adsorption rate (%) | 5 | 60 | 48 |

<Test Example 4: Evaluation of adsorption stability>

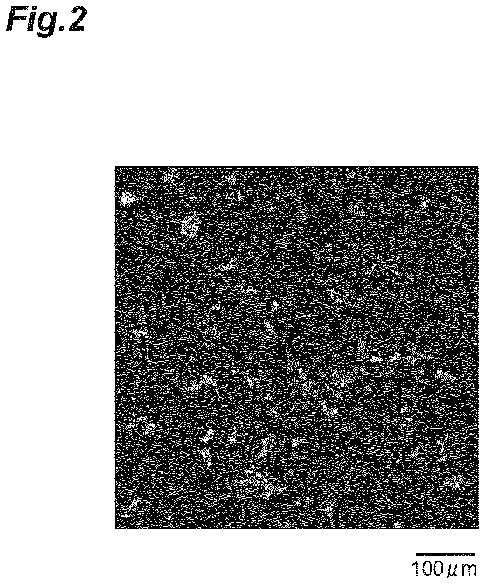

1 mg of sCMF was put into a 1.5 ml sample tube (WATSON, 131-7155C), and 1 ml of a solution containing Compound 2 was added thereto (eight samples were prepared). The mixture was stirred using ROTATOR (TAITEC, RT-50) at room temperature for 1 hour at a speed of 20 rpm. The mixture was centrifuged using a centrifugal separator (Eppendorf, minispin) at 3,500 rpm for 5 minutes, and the fluorescence intensity of 200 μl of each supernatant was measured using a spectrofluorometer (commercially available from JASCO Corporation, FP-8500). The obtained fluorescence intensity was applied to the calibration curve and the concentration of the fluorescent reagent in the supernatant was calculated. This was set as X. Each supernatant was aspirated, and 1 ml of PBS was added thereto. After 5, 20, 40, 60, 120, 300, 1,440, or 5,760 minutes, each supernatant was centrifuged using a centrifugal separator at 3,500 rpm for 5 minutes, and the fluorescence intensity of 200 μl of each supernatant was measured using a spectrofluorometer. The obtained fluorescence intensity was applied to the calibration curve, and the concentration of the fluorescent reagent in the supernatant was calculated. This was set as Y. The adsorption stability was evaluated according to adsorption stability index: formula of (X-Y)/X×100. FIG. 2 is a microscopic image of the test result after 5,760 minutes.

[Table 2]

| Time (min) | Adsorption stability index |
|---|---|
| 0 | 100 |
| 5 | 83 |
| 20 | 79 |
| 40 | 71 |
| 60 | 77 |
| 120 | 67 |
| 300 | 71 |
| 1,440 | 43 |
| 5,760 | 12 |

As shown in Table 2, it shows that Compound 2 was bound and/or adsorbed to sCMF even after 4 days (5,760 minutes).

<Test Example 5: Evaluation 1 of three-dimensional tissue construct using sCMF-FN>

5 mg of sCMF was added to 5 ml of 0.04% fibronectin (SIGMA, F2006-5MG) in 50 mM tris-HCl, and the mixture was stirred using ROTATOR (TAITEC, RT-50) at room temperature for 1 hour at a speed of 20 rpm. For stirring, immediately before mixing with cells, the stirring time ended. The mixture was centrifuged using a centrifugal separator (Eppendorf, minispin) under a condition of 3,500 rpm for 5 minutes. The supernatant was aspirated, and 300 μL of a culture medium was added thereto. The sCMF produced according to these operations was called sCMF-FN.

$1.0\times10^6$ cells of NHDF (Lonza, CC-2509) and $5.0\times10^5$ cells of HUVEC (Lonza, C2517A) were mixed with the above sCMF-FN, and seeded in a 24-well insert (costar, 3470-clear).

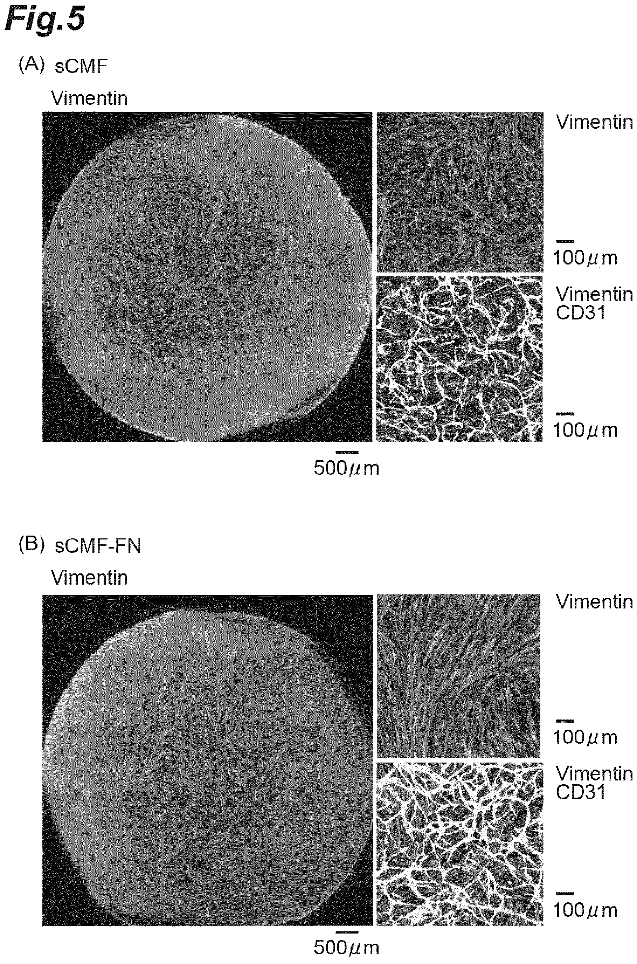

The sample was centrifuged at 1,100 g for 15 minutes to precipitate sCMF-FN and cells, and cultured in 1 mL of a mixed culture medium (DMEM (08489-45 commercially available from Nacalai Tesque, Inc.) 50%/EBM-2 (Lonza, CC-3202 commercially available from Nacalai Tesque, Inc.,) 50%) for 1 day. The resulting cultured product was transferred to a 6-well plate (IWAKI, 3810-006), and cultured in 12 mL of a mixed culture medium for 6 days. Immunostaining was performed using CD31 (Dako, M0823) and Alexa647 (Invitrogen, A21235), the fluorescence was observed using a confocal quantitative image cytometer CQ (YOKOGAWA), and the diameter of the capillary vessels was measured from the MIP image using image analysis software (ImageJ, NIH). In order to measure the diameter of the capillary vessels, a line was drawn according to the diameter manually, not automatically, and the length thereof was measured. An experiment was performed in the same procedures using sCMF in place of sCMF-FN. FIG. 5 shows the observation results of the produced three-dimensional tissue constructs. When the three-dimensional tissue construct was produced using sCMF-FN, compared to the three-dimensional tissue construct produced using sCMF, it was possible to obtain a three-dimensional tissue construct in which a thicker vascular network was formed.

<Test Example 6: Evaluation 2 of three-dimensional tissue construct using sCMF-FN>

A three-dimensional tissue construct was produced in the same manner as in Test Example 5. Using the three-dimensional tissue construct, CD31 immunostained and toluidine blue stained sections were prepared by Applied Medical Service Co., Ltd.

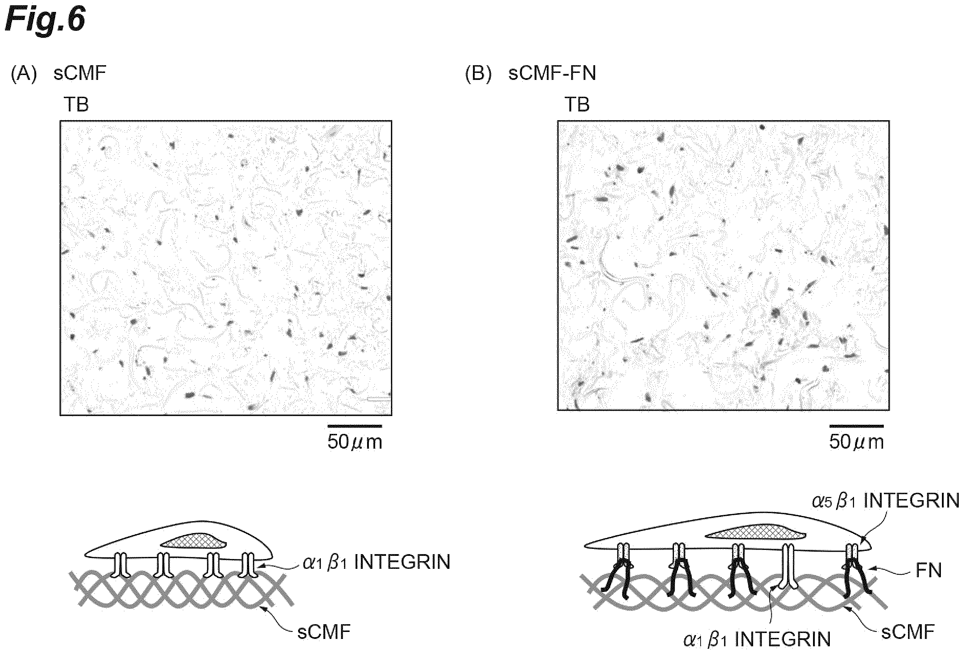

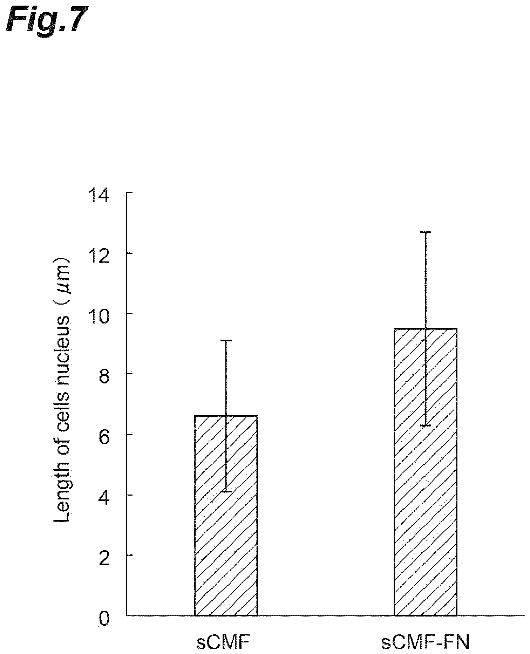

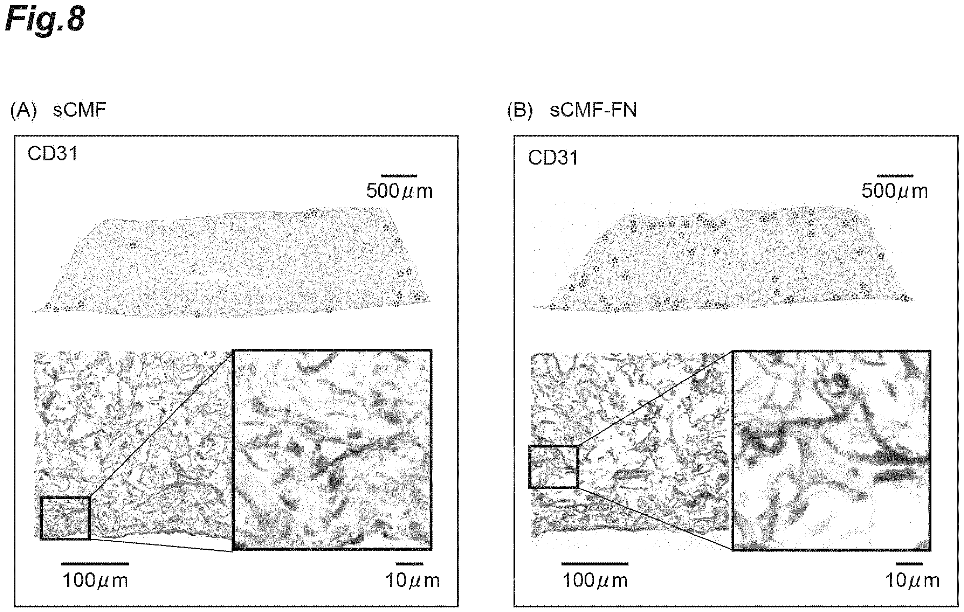

The prepared section image was captured using a microscope. For the toluidine blue (TB) stained section, the major axis of nuclei stained in blue was measured at N=20 using image analysis software (ImageJ, NIH). For the CD31 immunostained section, the number of parts in which the CD31 immunostained part (brown) was connected in a circle and the interior was hollow (white) was measured from the entire image. FIG. 6 and FIG. 8 show the observation results of the produced three-dimensional tissue constructs. FIG. 7 shows the measurement results of the length of nuclei in the three-dimensional tissue construct. When the three-dimensional tissue construct was produced using sCMF-FN, compared to the three-dimensional tissue construct produced using sCMF, it was possible to obtain a three-dimensional tissue construct in which a thicker lumen was formed. This was an effect that was not expected when fibronectin contributing to adhesiveness between cells was mixed and was an unexpected effect that was exhibited when fibronectin was bound or adsorbed to the fibrillated extracellular matrix.

## Claims

1. An extracellular matrix-containing composition, comprising a fragmented extracellular matrix component and a compound bound or adsorbed to the fragmented extracellular matrix component.

2. The extracellular matrix-containing composition according to claim 1,
   wherein the fragmented extracellular matrix component comprises a fragmented collagen component.

3. The extracellular matrix-containing composition according to claim 1 or 2,
   wherein the average length of the fragmented extracellular matrix component is 100 nm or more and 200 $\mu$m or less.

4. The extracellular matrix-containing composition according to any one of claims 1 to 3,
   wherein the compound is at least one selected from the group consisting of heparan sulfate, chondroitin sulfate, and fibronectin.

5. A method for producing an extracellular matrix-containing composition, comprising
   a process of bringing a fragmented extracellular matrix component into contact with a compound that is able to be bound or adsorbed to the fragmented extracellular matrix component.

6. The method for producing an extracellular matrix-containing composition according to claim 5,
   wherein the fragmented extracellular matrix component is obtained by fragmenting an extracellular matrix component in an aqueous medium.

7. The method for producing an extracellular matrix-containing composition according to claim 5 or 6,
   wherein the fragmented extracellular matrix component contains a fragmented collagen component.

8. A method for producing a three-dimensional tissue construct, comprising:

   a first process in which the extracellular matrix-containing composition according to any one of claims 1 to 4 is brought into contact with cells in an aqueous medium; and
   a second process in which the cells in contact with the extracellular matrix-containing composition are cultured.

9. The method for producing a three-dimensional tissue construct according to claim 8, comprising
   a process in which, in the aqueous medium, the fragmented extracellular matrix component, a compound bound or adsorbed to the fragmented extracellular matrix component, and the cells are precipitated, after the first process and before the second process.

10. The method for producing a three-dimensional tissue construct according to claim 8 or 9,
    wherein the first process is performed after a layer of the cells is formed in the aqueous medium.

11. The method for producing a three-dimensional tissue construct according to any one of claims 8 to 10,
    wherein the cells contain extracellular matrix producing cells.

12. The method for producing a three-dimensional tissue construct according to any one of claims 8 to 11,
    wherein the cells are one or more types of cells selected from the group consisting of vascular endothelial cells, cancer cells, cardiomyocytes, smooth muscle cells, and epithelial cells.

13. The method for producing a three-dimensional tissue construct according to any one of claims 8 to 12,
    wherein a total content of the fragmented extracellular matrix component and the compound based on a total mass of the fragmented extracellular matrix component, the compound and the cells is 10 mass% or more and 90 mass% or less.

14. A three-dimensional tissue construct containing cells and the extracellular matrix-containing composition according to any one of claims 1 to 4,
    wherein at least some of the cells are in contact with the fragmented extracellular matrix component.

# Fig.1

| HA | CS | HS | FN |
|---|---|---|---|
| Fluorescein | Fluorescein | Fluorescein | Rhodamine |

100μm ／ 100μm ／ 100μm ／ 100μm

| Fluorescein + Ph | Fluorescein + Ph | Fluorescein + Ph | Rhodamine + Ph |
|---|---|---|---|

100μm ／ 100μm ／ 100μm ／ 100μm

**Fig.2**

100μm

# Fig.3

(A) sCMF

CD31

500μm

100μm          100μm

(B) sCMF-FN

CD31

500μm

100μm          100μm

# Fig.4

*Fig.5*

(A) sCMF

Vimentin

Vimentin

100μm

Vimentin
CD31

100μm

500μm

(B) sCMF-FN

Vimentin

Vimentin

100μm

Vimentin
CD31

100μm

500μm

# Fig.6

(A)  sCMF

TB

50μm

(B)  sCMF-FN

TB

50μm

$\alpha_1\beta_1$ INTEGRIN

sCMF

$\alpha_5\beta_1$ INTEGRIN

FN

$\alpha_1\beta_1$ INTEGRIN

sCMF

EP 3 950 710 A1

# Fig.7

# Fig.8

(A) sCMF

(B) sCMF-FN

EP 3 950 710 A1

<table>
<tr><td colspan="2" align="center">INTERNATIONAL SEARCH REPORT</td><td>International application No.<br>PCT/JP2020/013436</td></tr>
</table>

**A. CLASSIFICATION OF SUBJECT MATTER**
Int.Cl. C07K14/78(2006.01)i, C12M3/00(2006.01)i, C12N5/071(2010.01)i, C12N5/09(2010.01)i
FI: C12N5/071, C12N5/09, C07K14/78, C12M3/00A
According to International Patent Classification (IPC) or to both national classification and IPC

**B. FIELDS SEARCHED**

Minimum documentation searched (classification system followed by classification symbols)
Int.Cl. C07K14/78, C12M3/00, C12N5/071, C12N5/09

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580(JDreamIII), WPIDS/WPIX(STN), AGRICOLA(STN), FSTA(STN), TOXCENTER(STN)

**C. DOCUMENTS CONSIDERED TO BE RELEVANT**

| Category* | Citation of document, with indication, where appropriate, of the relevant passages | Relevant to claim No. |
|---|---|---|
| A | WO 2018/186185 A1 (NIKKISO CO., LTD.) 11.10.2018 (2018-10-11), entire text, all drawings | 1-14 |
| A | 西宏基, 他, コラーゲンマイクロファイバーを用いた iPS 細胞由来心筋細胞組織の構築, 高分子学会予稿集 第 66 回高分子年次大会, 29 May 2017, vol. 66, no. 1, 2Pa097, entire text, all drawings, (NISHI, Koki et al., Polymer Preprints, Japan 66th SPSJ Annual Meeting), non-official translation (Construction of iPSC-derived cardiomyocytes tissue using collagen microfiber) | 1-14 |

☒ Further documents are listed in the continuation of Box C.　　☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>02.06.2020 | Date of mailing of the international search report<br>16.06.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| | International application No. |
|---|---|
| | PCT/JP2020/013436 |

C (Continuation).   DOCUMENTS CONSIDERED TO BE RELEVANT

| Category* | Citation of document, with indication, where appropriate, of the relevant passages | Relevant to claim No. |
|---|---|---|
| A | 米田美咲，他，コラーゲンマイクロファイバーによる高濃度細胞外マトリックスと毛細血管網を有する三次元間質組織体の構築，高分子学会予稿集 第66回高分子討論会, 20 September 2017, vol. 66, no. 2, 2Q10, entire text, all drawings, (KOMEDA, Misaki et al., Polymer Preprints, Japan 66th Symposium on Macromolecules), non-official translation (Construction of three-dimensional interstitial tissue with high concentration extracellular matrix and capillary network by collagen microfiber) | 1-14 |
| A | 加藤菜津子，他，コラーゲンマイクロファイバーを用いた高密度ECMを有する三次元ヒトがん-間質組織体の構築，高分子学会予稿集 第66回高分子討論会, 20 September 2017, vol. 66, no. 2, 2Q11, entire text, all drawings, (KATO, Natsuko et al., Polymer Preprints, Japan 66th Symposium on Macromolecules), non-official translation (Construction of three-dimensional human cancer-stromal tissue with high-density ECM using collagen microfiber) | 1-14 |
| A | 松崎典弥，他，コラーゲンマイクロファイバーを用いた沈殿培養による高密度ECMを有する3D-結合組織の構築，高分子学会予稿集 第66回高分子討論会, 20 September 2017, vol. 66, no. 2, 3N02, entire text, all drawings, (MICHIYA, Matsusaki et al., Polymer Preprints, Japan 66th Symposium on Macromolecules), non-official translation (Construction of 3D-connective tissue with high density ECM by precipitation culture using collagen microfiber) | 1-14 |
| P, A | WO 2019/208831 A1 (TOPPAN PRINTING CO., LTD.) 31.10.2019 (2019-10-31), entire text, all drawings | 1-14 |
| P, A | WO 2019/208832 A1 (TOPPAN PRINTING CO., LTD.) 31.10.2019 (2019-10-31), entire text, all drawings | 1-14 |

Form PCT/ISA/210 (second sheet) (January 2015)

| INTERNATIONAL SEARCH REPORT<br>Information on patent family members | International application No.<br>PCT/JP2020/013436 |
|---|---|

```
WO 2018/186185 A1  11.10.2018    (Family: none)

WO 2019/208831 A1  31.10.2019    (Family: none)

WO 2019/208832 A1  31.10.2019    (Family: none)
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012115254 A
- WO 2015072164 A
- WO 2016027853 A
- WO 2017146124 A
- WO 2018143286 A

**Non-patent literature cited in the description**

- **M. C. ERAT et al.** *Proc. Natl. Acad. Sci. USA,* 2009, vol. 106, 4195
- **Y. TATARA et al.** *Glycobiology,* 2015, vol. 25, 557
- **M. SHAWN et al.** *Proc. Natl. Acad. Sci. USA,* 1998, vol. 95, 7275